# EUROPÄISCHE PATENTSCHRIFT

(11) **EP 3 325 952 B1**
(45) Veröffentlichungstag und Bekanntmachung des Hinweises auf die Patenterteilung: **29.07.2020**
(21) Anmeldenummer: 16756567.0
(22) Anmeldetag: 11.07.2016
(51) Int. Cl.: G01N 27/30, G01N 27/416, G01N 33/18

(54) **ELEKTROCHEMISCHE MESSZELLE ZUR MESSUNG DES GEHALTES VON CHLORVERBINDUNGEN IN WASSER**
ELECTROCHEMICAL MEASURING CELL FOR MEASURING THE CONTENT OF CHLORINE COMPOUNDS IN WATER
CELLULE DE MESURE ÉLECTROCHIMIQUE POUR LA MESURE DE LA TENEUR DE L'EAU EN COMPOSÉS CHLORÉS

(30) Priorität: 22.07.2015 DE 102015111849
(43) Veröffentlichungstag der Anmeldung: 30.05.2018
(73) Patentinhaber: Kuntze Instruments GmbH, 40668 Meerbusch (DE)
(72) Erfinder: KUNTZE, Verena, 40549 Düsseldorf (DE)
(74) Vertreter: Sroka, Peter-Christian
(86) Internationale Anmeldenummer: PCT/DE2016/100306
(87) Internationale Veröffentlichungsnummer: WO 2017/012607

(56) Entgegenhaltungen:
- DE-A1- 10 322 894
- US-A- 5 944 969
- SCHIAVON G ET AL: "AMPEROMETRIC MONITORING OF SULPHUR DIOXIDE IN LIQUID AND AIR SAMPLES OF LOW CONDUCTIVITY BY ELECTRODES SUPPORTED ON ION-EXCHANGEMEMBRANES", ANALYST, LONDON, GB, Bd. 116, Nr. 8, 1. August 1991 (1991-08-01), Seiten 797-801, XP000881279, DOI: 10.1039/AN9911600797
- RENE KNAKE ET AL: "Amperometric sensing in the gas-phase", ANALYTICA CHIMICA ACTA, ELSEVIER, AMSTERDAM, NL, Bd. 549, 6. Juli 2005 (2005-07-06), Seiten 1-9, XP007922081, ISSN: 0003-2670, DOI: 10.1016/J.ACA.2005.06.007 [gefunden am 2005-07-06]

## Beschreibung

Die Erfindung betrifft eine elektrochemische Messzelle gemäß dem Oberbegriff des Patentanspruchs 1.

Derartige elektrochemische Messzellen sind u.a. beschrieben in der EP 0 740 149 B1, der EP 0 563 690 A1, der DE 195 15 392 C2 und der DE 103 22 894 A1.

So offenbart die DE 195 15 392 C2 eine Elektrolyten-Membran-Kombination für eine elektrochemische Messzelle für Stoffe in Flüssigkeiten. Chlorverbindungen, die mit in Elektrolyt befindlichen Reagenzien reagieren, sind nicht offenbart. Die Gegenelektrode befindet sich innerhalb des Elektrolytraums. Es fehlen Angaben über die Messelektrode.

Bei diesen bekannten Messzellen handelt es sich um sogenannte membrangedeckte Messzellen mit einer hydrophilen, flüssigkeitsdurchlässigen Membran, die den Elektrolytraum bzw. den darin befindlichen Elektrolyten gegenüber der zu untersuchenden Flüssigkeit, insbesondere Wasser, abgrenzt.

Schiavon G et al: "Amperometric Monitoring of Sulphur Dioxide in Liquid and Air samples of low Conductivity by elektrodes supported on Ion-Exchangemembranes" offenbart keinen Chlorverbindungssensor, keine außenliegende Gegenelektrode und keine Spezifikation der Arbeitselektrode. Darüber hinaus wird kein Reagenz, welches mit Chlor reagiert, beschrieben.

"Rene Knake et al: "Amperomatic sensing in the gas-phase" ist ein Übersichtsartikel für amperometrische Sauerstoffsensoren in einer Lösung mit einer porösen Membran über einer massiven Arbeitselektrode. Die Gegenelektrode befindet sich innerhalb des Elektrolytraums.

Die bekannten membrangedeckten Sensoren haben einige Nachteile
- Die zu messenden Verbindungen müssen durch die Membran in den Elektrolytraum diffundieren. Das ist ein langsamer Prozess, die Signalstärke sinkt stark und die Ansprechzeit steigt stark im Vergleich zu offenen Elektrodensystemen. Außerdem kann auch in umgekehrter Richtung eine Diffusion der im Elektrolyt gelösten Salze in das zu untersuchende Wasser stattfinden, was die Lebensdauer der Messzelle beträchtlich verkürzen kann.
- Wegen des langsamen Diffusionsprozesses muss die Membran möglichst dünn sein. Das macht sie mechanisch empfindlich. Da der Elektrolyt sich durch Messung und Lagerung verbraucht, muss er regelmäßig gewechselt werden. Dazu muss eine Membrankappe abgeschraubt, geleert, gespült, gefüllt und wieder aufgeschraubt werden, wobei für einen Druckausgleich gesorgt werden muss. Diese Handhabung birgt stets die Gefahr, dass der Betreiber die Membran dabei beschädigt und den Sensor damit unbrauchbar macht.
- Da auch hinter der Membran ein Stofftransport nur durch Diffusion möglich ist, hat der Abstand zwischen Membran und Messelektrode einen entscheidenden Einfluss auf die Signalstärke. Dieser Abstand kann aber bei Druckschwankungen oder Änderungen in der Anströmung variieren, so dass das Mess-Signal solche Druck- oder Strömungsänderungen mit anzeigt.
- Die auf dem Markt verfügbaren Sensoren haben sehr kleine Elektroden mit entsprechend schwachen Signalen, die eine Vorverstärkung, also eine in den Sensor eingebaute Elektronik erfordern, was sich negativ auf die Herstellungskosten auswirkt. Um dem zu begegnen, wird nicht der Sensor als Verbrauchsmaterial angesehen, sondern nur die Membran und der Elektrolyt. In der Praxis wird von manchen Herstellern angeboten, die Elektroden des Sensors auf Wunsch gegen Entgelt aufzuarbeiten.
- Membransensoren mit hydrophilen, flüssigkeitsdurchlässigen Membranen enthalten verschiedene Kunststoffmaterialien, Verklebungen und Dichtungen, die zum Teil empfindlich auf Wasserinhaltsstoffe reagieren, z.B. auf Tenside.

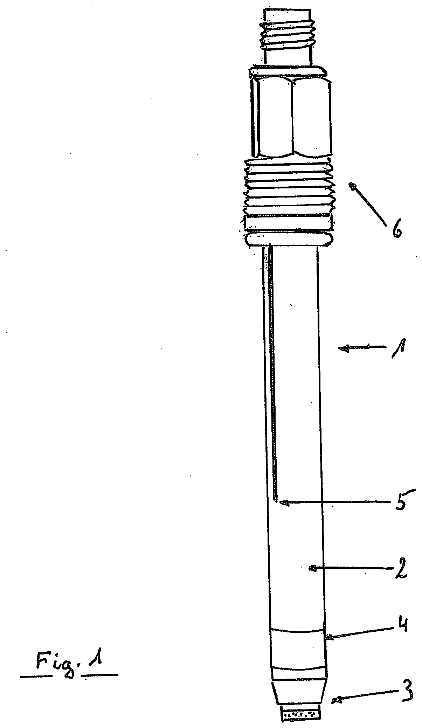

Eine in der US 4,707,242 A beschriebene elektrochemische Zelle enthält zwei Arbeitselektroden 20, 22, eine Referenzelektrode 34 und eine Gegenelektrode 28 und dient zur quantitativen Messung von schädlichen Gasen. Die Referenzelektrode 34 und die auf einer porösen Membran 30 angebrachte Gegenelektrode befinden sich in einem Elektrolytraum 14, in den das zu untersuchende Gas eingepumpt wird Die Arbeitselektroden 20, 22 sind gemäß Figur 3 als eine erste Einheit auf einer eine zweite Einheit bildenden porösen Gasdiffusionsmembran 24 angebracht. Die zweite Arbeitselektrode 22 umgibt die erste Arbeitselektrode 20. Die erste Arbeitselektrode 20 ist durch Mischen eines geeigneten Katalysators 40 mit einer Polytetrafluorethylendispersion hergestellt. Der Katalysator 40 kann aus Platin bestehen. Die zweite Arbeitselektrode 22 hat einen katalytischen Bereich 50, der aus Platin bestehen kann. Diese bekannte elektrochemische Zelle ist nicht zur Messung des Chlorverbindungen in Wasser geeignet.

In der US 5,326,449 A ist ein Sensor für die elektrochemische Analyse eines katalysierten Reagenz in Lösung beschrieben. Dieser Sensor hat eine Verbundmembran 140 aus einem katalytisch imobilisierten Protein zur Umwandlung des zu messenden Reagenz in einen elektrisch messbaren Wert. Die Verbundmembran enthält eine poröse Membran 142 aus einem synthetischen Polymermaterial in einer dünnen flexiblen Schicht. In die das Protein enthaltende Membran ist mindestens eine Sperrmembran 148 teilweise eingebettet, die als Schutzmembran zwischen der porösen Membran und dem Analyten liegt. Gemäß Spalte 7, Zeilen 15 ff. haben die porösen Membranelemente eine Porengröße im Bereich von etwa 0,01 mikron bis etwa 10 mikron, vorzugsweise 0,1 bis etwa 2 mikron. Dieser bekannte Sensor ist nicht geeignet zur Messung des Gehaltes von Chlorverbindungen in Wasser.

In der US 4,552,013 A ist ein Flüssig-Chromatografie-Gerät mit einer coulometrischen Zelle 23 beschrieben, die mindestens eine Arbeitselektrode, mindestens eine Referenzelektrode und mindestens eine Gegenelektrode enthält. Die Arbeitselektrode 54 enthält eine Materialpackung 68 aus pulverigem Platinmaterial, mit einer mittleren Korngröße zwischen etwa 2 bis 3 mikron und etwa 400 mikron. Die Materialpackung 68 befindet sich zwischen zwei porösen Membranen oder Fritten aus z.B. Glas, Glasfaser, Polypropylen, porösem Teflon oder dergleichen und ist in einem Strömungskanal 46 für die zu untersuchende Probelösung angeordnet, die durch die Arbeitselektrode 54 und damit durch die Materialpackung 68 aus pulverigem Platinmaterial strömt. Die Zelle 23 hat keinen Elektrolytraum. Bei der Herstellung der Zelle 23 wird das trockene, pulverige Platinmaterial auf eine der beiden porösen Membranen (Fritten) geschüttet und dann mit der zweiten Membran überdeckt. Der Zweck der bekannten Zelle besteht darin, vor der Chromatografiekolonne 28, aus einer Probelösung störende Inhaltsstoffe wie beispielsweise Sauerstoff zu entfernen. In der US 4,552,013 ist ohne genauere Spezifizierung zum Ausdruck gebracht, dass die bekannte Zelle auch zu Messzwecken benutzt werden kann.

In der US 5,944,969 ist ein elektrochemischer Sensor beschrieben, der mindestens zwei elektrochemisch aktive Elektroden, ein nicht wässriges Elektrolytsystem und eine Diffusionssperrmembran enthält, durch die der Analyt in seiner Gasphase beweglich ist, aber durch die das nicht wässrige Elektrolytsystem im wesentlichen nicht durchlässig ist. Die Diffusionssperrmembran ermöglicht es damit dem Analyt in seiner Gasphase, in den Sensor einzutreten, während im wesentlichen verhindert wird, dass das nicht wässrige Elektrolytsystem aus dem Sensor nach außen austreten kann. Für den Fall eines Chlorsensors wird als elektrochemische Oberfläche der Referenzelektrode Platin genannt, das in Form einer Platinpulver enthaltenden Tinte auf einen porösen Film aufgetragen worden ist. Dieser Film hat eine Dicke von etwa 1 bis 10 mil (= 0,0254 mm bis 0,254 mm).

Die EP 0 563 690 A1 offenbart eine elektrochemische Messzelle zum Nachweis von auch an organische Trägermaterialien gebundenem Chlor, enthaltend
- einen einen Elektrolyten aufnehmenden Elektrolytraum mit einem Elektrolyten und einem mit Chlorverbindungen reagierenden Reagenz,
- eine den Elektrolytraum begrenzende Messelektrode,
- eine mit Abstand von der Messelektrode liegende mikroporöse Membran, die den Durchgang von elektrischen Ladungsträgern erlaubt,
- eine innerhalb des Elektrolytraumes angeordnete Referenzelektrode und
- eine Gegenelektrode, wobei
- die Gegenelektrode außerhalb des Elektrolytraums angeordnet ist.

Die zu detektierende Substanz, z.B. Chlorderivate, diffundiert hinter der mikroporösen Membran durch eine mit einem Loch versehene starre (aber nicht explizit als porös definierte) Messelektrode in den Elektrolytraum. Diese Druckschrift enthält keine Angaben zum Aufbau der Messelektrode.

Der Erfindung liegt die Aufgabe zugrunde, eine elektrochemische Messzelle zur Messung des Gehaltes an Chlorverbindungen in Wasser zu schaffen, bei der auf eine flexible, möglichst dünne, hydrophile Membran verzichtet werden kann, um die oben geschilderten Gefahren bzw. Nachteile bei der Handhabung und der Reinigung der Messzelle zu vermeiden, ohne das spätere Messergebnisse negativ beeinträchtigt werden.

Das mit der Erfindung zu lösende technische Problem besteht weiterhin darin, eine Messzelle mit einer niedrigen Ansprechzeit zu schaffen.

Diese Aufgabe wird erfindungsgemäß durch die Merkmale des Kennzeichens des Patentanspruchs 1 gelöst, wobei die gleichzeitig die Messelektrode bildende poröse Platinmembran dazu dient, dass das zu untersuchende Wasser nicht in den den Elektrolyt aufnehmenden Elektrolytraum eindringt, wobei dadurch, dass die Reaktion nur in den Poren der Platinmembran stattfindet, die entstandenen Reaktionsprodukte direkt bei ihrer Entstehung gemessen werden.

Bei der erfingungsgemäßen Messzelle handelt es sich um eine solche zur amperometrischen, potentiostatischen Messung von Chlorverbindungen.

Bevorzugte Ausführungsformen der Erfindung sind in den Unteransprüchen behandelt.

Bei der erfindungsgemäßen Messzelle sind die Membran und die Messelektrode in einer Komponente vereint. Die mikroporöse Platinmembran, trennt den Elektrolytraum von dem zu untersuchendem Wasser, so dass aktive Inhaltsstoffe des Elektrolyten und der zu messenden Stoffe aus dem Wasser sich in den Poren der Messelektrode treffen, dort reagieren und das gebildete Reaktionsprodukt erfasst wird.

Das Vereinen der beiden Funktionen (Membran und Messelektrode) zu einem Bauteil eliminiert die bisherigen Probleme des Druckeinflusses und des Durchflusseinflusses und optimiert die Diffusionsproblematik. Da es sich um eine starre Komponente handelt, die fest eingebaut ist und nicht ausgewechselt werden muss, ist die neue Messzelle mechanisch unempfindlich und birgt nicht die Gefahr, dass der Benutzer durch fehlerhaften Austausch von Verschleißteilen die Messung gefährdet.

Da der Elektrolyt nur über die Poren der starren, porösen Platinmembran mit dem Wasser in Kontakt steht, ist ein Auswaschen des Elektrolyten weitgehend ausgeschlossen.

Die Kontaktfläche Elektrolyt/Wasser in den Poren der porösen starren Platinmembran ist um ein Vielfaches grösser als bei handelsüblichen Membransensoren, das erhaltene Signal ist stark und erfordert keine Vorverstärkung. Die Messzelle kann daher in Form der für wasseranalytische Messzellen gebräuchlichen 12mm-Glassensoren gebaut werden und über sein typisches PG13,5-Gewinde in typische pH- oder Redox-Armaturen eingebaut werden.

Die Messzelle ist aus Glas gefertigt und daher unempfindlich gegen die meisten Wasserinhaltsstoffe, wie z. B. Tenside.

Die vereinte Ausführung von Membran und Messelektrode in Form eines porösen Platins bietet den Vorteil, dass die Messzelle langzeitig sauber und aktiv bleibt. Die erfindungsgemäße Messzelle kann problemlos für Anwendungen eingesetzt werden, bei denen über längere Zeiträume kein Desinfektionsmittel vorhanden ist.

Platinelektroden werden zum Beispiel eingesetzt in Brennstoffzellen. Dabei wird aber nur die Oberflächenvergrösserung genutzt, um Materialkosten zu senken. Die bekannten Platinelektroden dienen auch nicht zur insbesondere amperometrischen Messung sondern nur zum Stoffumsatz.
Poröse Platinelektroden, die zur Messung genutzt werden und bei denen Stoffe durch die Membranporen hindurchtreten, findet man zum Beispiel in Lambdasonden, die zur Regelung der Luftzufuhr in Abgaskatalysatoren eingesetzt werden. Dabei handelt es sich allerdings nicht um amperometrische sondern um Redox-Messungen.

Die Messzelle besteht gemäß der Zeichnung aus einem 12mm-Glasgehäuse 1, an dessen unterem Ende die Messelektrode 3 in Form einer starren mikroporösen Platinmembran mit einer Porengröße von 0,15 µm bis 0,25 µm, vorzugsweise 0,2 µm, befestigt ist. Im Innern des Gehäuses 1 befinden sich die Referenzelektrode 5 und der Elektrolytraum 2. Am schaftartigen Gehäuse 1 ist ein Platinring als Gegenelektrode 4 angebracht. Am oberen Ende des Gehäuses befindet sich ein Anschlusskopf 6 zum Anschließen an eine Messwert-Auswertungseinheit.

Die poröse Platinmembran der Messelektrode 3 führt zu einem innigen Kontakt zwischen dem Elektrolyt und dem zu messendem Stoff in den Poren der Platinmembran 3. Das entstehende Produkt wird an der Messelektrode 3 erfasst und dabei reduziert. Das führt zu einem Stromfluss, der proportional zur Konzentration des zu messenden Stoffes ist.

Durch die starre, poröse Platinmembran wird eine sensitivere und physikalisch stabilere Messelektrode zur Detektion von Chlorverbindungen erhalten. Darüber hinaus ist der Aufbau der erfindungsgemäßen Messzelle vereinfacht, da die Messelektrode und die Membran einstückig sind.

## Patentansprüche

1. Elektrochemische Messzelle zur Messung des Gehalts von Chlorverbindungen in Wasser, enthaltend
- einen Elektrolyten aufnehmenden Elektrolytraum (2) mit einem darin aufgenommenen Elektrolyten und einem sich im Elektrolyten befindlichen Reagenz, welches mit den sich im Wasser befindlichen Chlorverbindungen reagiert,
- eine den Elektrolytraum (2) begrenzende Messelektrode (3),
- eine innerhalb des Elektrolytraumes (2) angeordnete Referenzelektrode (5)
- eine Gegenelektrode (4),
**dadurch gekennzeichnet, dass**
- die Messelektrode (3) eine starre, poröse Platinmembran mit einer Porengröße von 0,15 µm bis 0,25 µm und mit einer Dicke von 0,5 mm +/- 0,05 mm ist,
- dass ein physikalischer und ein elektrischer Kontakt zwischen dem zu untersuchenden Wasser und dem Elektrolyten nur innerhalb der Platin-Membranporen stattfindet,
- die Gegenelektrode (4) den Elektrolytraum (2) ringförmig umgibt.

2. Elektrochemische Messzelle nach Anspruch 1, **dadurch gekennzeichnet, dass** die Porengröße 0,2 µm beträgt.

3. Elektrochemische Messzelle nach Anspruch 1 oder 2, **dadurch gekennzeichnet, dass** die Gegenelektrode (4) Platin enthält.

4. Elektrochemische Messzelle nach einem der Ansprüche 1 bis 3, **dadurch gekennzeichnet, dass** die Gegenelektrode (4) ein Platinring ist.

## Claims

1. Electrochemical measuring cell for measuring the content of chlorine compounds in water
- containing an electrolyte space (2) containing an electrolyte and a reagent within the electrolyte, which reagent can react with the chlorine compounds contained in the water,
- a measuring electrode (3) delimiting the electrolyte space (2),
- a reference electrode (5) which is arranged within the electrolyte space (2),
- a counter electrode (4)
**characterised in that**
- the measuring electrode (3) is rigid porous platinum membrane having a pore size of 0,15 µm to 0,25 µm and a thickness of 0,5 mm +/- 0,05 mm
- a physical and electrical contact takes place between the water to be examined and the electrolyte only within the pores of the platinum electrode,
- the counter electrode (4) surrounds the electrolyte space (2) ringlike.

2. Electrochemical measuring cell according to claim 1, **characterised in that** the pore size is 0,2µm.

3. Electrochemical measuring cell according to claim 1 or 2, **characterised in that** the counter electrode (4) contains platinum.

4. Electrochemical measuring cell according to one of claims 1 or 3, **characterised in that** the counter electrode (4) is a platinum ring.

## Revendications

1. Capteur électrochimique pour le mesurage de la teneur des composés du chlore dans l'eau, contenant
- un électrolyte espace (2) contenant un électrolyte contenant un réactif, qui peut réagir avec les composés du clore étant dans l'eau,
- une électrode de mesure (3) délimitant l'espace (2),
- une électrode de référence (5) qui est placé dans l'espace (2)
- une contre-électrode (4)
**caractérisée en ce que**
- l'électrode de mesure (3) est une membrane en platine rigide et poreuse avec une grosseur de pore entre 0,15µm et 0,25µm et une épaisseur de 0,5 mm +/-0,05 mm,
- un contact physique et électrique entre l'eau à analyser et l'électrolyte a lieu seulement dans les pores de la membrane en platine,
- la contre-électrode (4) entoure l'espace (2) annulairement.

2. Capteur électrochimique selon la revendication 1, **caractérisée en ce que** la grosseur de pore est 0,2µm.

3. Capteur électrochimique selon la revendication 1 ou 2, **caractérisée en ce que** la contre-électrode contient platine.

4. Capteur électrochimique selon une des revendications 1 à 3, **caractérisée en ce que** la contre-électrode est un anneau en platine.
